# EUROPEAN PATENT APPLICATION

(11) **EP 2 236 093 A2**
(43) Date of publication of application: **06.10.2010**
(21) Application number: 10250711.8
(22) Date of filing: 31.03.2010
(51) Int. Cl.: A61B 17/04

(54) **Suture management port**

(30) Priority: 01.04.2009 US 165623 P; 17.03.2010 US 725672
(71) Applicant: Tyco Healthcare Group LP, North Haven, CT 06473 (US)
(72) Inventor: Carter, Sally, Wallingford, CT 06492 (US); Barnes, Andrew, Naugatuck, CT 06770 (US)
(74) Representative: Soames, Candida Jane

(57) **Abstract**

There are disclosed various embodiments of auxiliary and detachable caps for managing the passage of suture materials through a surgical cannula. The disclosed suture management caps (50) generally include one or more frame members (52) defining a central opening for the passage of suture material (56,58) and surgical instruments. The frame members (52) include one or more grasping members (60-70) for temporarily securing a length of suture material (56,58). A plurality of cutouts (72-78) is also provided in the rim of the frame (52) defining the central opening (54) for maintaining the lengths of suture material (56,58) in a spaced apart relationship.

## Description

### CROSS REFERENCE TO RELATED APPLICATION

The present application claims the benefit of and priority to U.S. Provisional Application Serial No. 61/165,623 filed on April 1, 2009, the entire contents of which are incorporated herein by reference.

### BACKGROUND

### Technical field

The present disclosure relates to auxiliary caps and seals for use with surgical cannula ports. More particularly, the present disclosure relates to auxiliary cannula caps incorporating suture management features for organized control of multiple sutures passing into a surgical cannula.

### Background Of Related Art

Various surgical procedures are often accomplished in a relatively non-invasive manner. A small slit or incision is made through the skin of a patient and a small access tube or cannula port is inserted through the incision. Surgical instruments are then inserted through the cannula port and the surgery performed. The cannula port generally includes a housing and an elongate tube extending from the housing. A valve is provided within the housing to seal the housing in the absence of a surgical instrument. An opening is provided at the proximal end of the housing to receive the surgical instruments and includes a seal having a smaller opening to seal about the surface of a surgical instrument inserted therethrough. The elongate tube is positioned through the incision and the surgical instruments are inserted into the opening and seal and into the body of the patient to perform the surgery. Often it is desirable to use a variety of differing diameter surgical instruments during the surgical procedure. The cannula port may be provided with alternative, detachable proximal caps having differing diameter openings and seals to accommodate the variety of surgical instruments.

Often, it is necessary to pass suture material through the cannula port to assist in the surgery. For example, it is often necessary to use the suture material to attach prosthetic materials or devices to surrounding tissue, to close interior wounds or incisions, etc. The suture material is manipulated by a surgical instrument inserted through the same or another cannula port. Multiple lengths of suture material may need to be passed through the cannula port simultaneously to properly perform the surgical procedure.

When two or more sutures pass simultaneously through the same cannula port there exists the potential for entanglement. For example, the sutures may slide toward each other around the surface of the proximal opening in the cannula port resulting in twisting or other entanglement about each other hindering or complicating the surgery. Furthermore, when one suture is being advanced or retracted through the opening it may grab or snag a second suture pulling the second suture with it.

Therefore, there exists a need for a detachable, auxiliary cap for use with the surgical cannula port which is capable of maintaining two or more sutures in separation. Further, there is a need for a detachable, auxiliary cap which can maintain tension on individual sutures to prevent one suture from inadvertently moving a second suture positioned through the cannula port.

### SUMMARY

There is disclosed a suture management cap for use with a surgical cannula. The suture management cap generally includes a frame releasably attachable to a surgical cannula and defining an opening and a grasping member located on the frame adjacent the opening for releasably securing a length of suture material. The grasping member is a notch formed in the frame. In one embodiment, the notch is v-shaped to temporarily secure a suture wedged therein.

The opening is a circular opening centrally located in the frame and defined by a rim of the frame. The rim includes at least one cut out for guiding a suture through the opening. The frame also includes a concave depression surrounding the opening for guiding surgical instruments towards opening.

In one embodiment, the frame includes a pair of opposed wings to facilitate securing the frame to a surgical cannula.

There is also disclosed a suture management cap assembly for use with a surgical cannula. The suture management cap assembly generally includes a suture management cap including a frame defining an opening and releasably attachable to a surgical cannula and at least one grasping member located on the frame adjacent the opening. The assembly further includes an auxiliary seal for receipt of a surgical instrument and a length of suture material.

In one embodiment, the auxiliary seal includes a first port for receipt of a surgical instrument and a second port for receipt of a length of suture material. The first port includes a circular seal having an opening for receipt of a surgical instrument. The second port includes a tri-slit seal for receipt of a surgical suture.

In an alternative embodiment of the suture management cap assembly, the auxiliary seal includes a first disk and a second disk, each of the first and second disks including a tri-slit seal. The first and second disks are positioned in overlapping relationship. The tri-slit seal of the first disk has a gap for receipt of a surgical instrument.

There is further disclosed a suture management cap assembly for use with a surgical cannula having a suture management cap including a frame defining an opening and releasably attachable to a surgical cannula and at least one grasping member located on the frame adjacent the opening. An auxiliary seal is provided in the frame for receipt of a surgical instrument and a length of suture material. The suture management cap assembly further includes a guide member for separating the surgical instrument from the length of suture material. The guide member includes a circular frame having a divider positioned within the circular frame to separate the surgical instrument from the length of suture material. The divider is positioned offset within the circular frame.

There is also disclosed a suture management cap for use with a surgical cannula including a frame releasably attachable to a surgical cannula and defining an opening. A grasping member is located in the frame and adjacent the opening. The grasping member includes a first side surface formed in the frame and a second side surface formed in the frame and facing the first side surface. At least one of the first and second side surfaces includes a vertically extending rib such that a length of suture material positioned between the first and second side surfaces is engaged by the vertically extending rib.

In one embodiment, the frame includes an inner frame member defining the opening and an outer frame member surrounding the inner frame member. The first side surface is formed on the outer frame member and the second side surface is formed on the inner frame member. The outer frame member includes an inwardly projecting arm and the inner frame member includes a pocket for receipt of the inwardly projecting arm of the outer frame member.
The first side surface is formed on the inwardly projecting arm of the outer frame member and includes a first pair of vertically extending ribs. The second side surface defines one side of the pocket and includes a second pair of vertically extending ribs.

In a specific embodiment, the first pair of vertically extending ribs is interleaved with the second pair of vertically extending ribs.

There is further disclosed a suture management cap for use with a surgical cannula which generally includes an outer frame member having a first central opening and at least one inwardly projecting arm extending into the central opening. The suture management cap additionally includes an inner frame member having a second central opening. The inner frame member has at least one pocket for receipt of the at least one inwardly projecting arm of the outer frame member. The suture management cap further includes a grasping member formed between the at least one inwardly projecting arm of the outer frame member and the at least one pocket of the inner frame member. The grasping member is provided to releasably secure a length of suture material positioned therein.

The at least one inwardly projecting arm includes a vertically extending rib and the inner frame member includes a vertically extending rib formed in a side surface of the pocket.

In a specific embodiment, the inwardly projecting arm includes a first pair of vertically extending ribs and the inner frame member includes a second pair of vertically extending ribs formed in a side surface of the pocket and opposite to the pair of vertically extending ribs of the inwardly projecting arm. The first pair of vertically extending ribs is interleaved with the second pair of vertically extending ribs.

The inner frame member includes a rim defining the second central opening, the rim includes at least one cut out for guiding a length of suture material into the second central opening.

There is also disclosed a suture management cap for use with a surgical cannula which generally includes an inner frame member having a rim defining an opening for receipt of surgical instruments and an outer frame member positionable over the inner frame member. The outer frame member includes at least one slot. A grasping member is positioned between the inner frame member and the outer frame member and extends across the at least one slot such that a length of suture material extending through the at least one slot is releasably held by the grasping member. In one embodiment, the grasping member is a coil spring.

In one embodiment, the inner frame member has a shelf portion for supporting coil spring and a wall portion extending upwardly from the shelf portion. The outer frame member includes at least one inwardly projecting arm located adjacent the at least one slot and engageable with the coil spring to hold the coil spring against the inner frame member. The at least one inwardly projecting arm terminates in a hook configured to engage the coil spring.

The rim of the inner frame member includes at least one cutout for guiding a length of suture material toward the opening. The rim of the inner frame member may include two spaced apart cutouts for separating a pair of lengths of suture material. The inner frame member includes a concave portion surrounding the opening.

There is also disclosed a suture management cap for use with a surgical cannula which generally includes a circular inner frame member having a rim defining an opening for receipt of surgical instruments therethrough. A circular outer frame member is positioned over the inner frame member and includes a plurality of radially extending slots and a plurality of inwardly projecting arms located between the radially extending slots. A grasping member is positioned between the inner frame member and the outer frame member and extends across the radially extending slots such that a length of suture extending through one of the radially extending slots is releasably held by the grasping member. The grasping member is a length of coil spring formed into a circular configuration.

There is also disclosed a suture management cap the surgical instrument which generally includes a frame defining a central opening for passage of a surgical instrument. The frame includes a plurality of pockets surrounding the central opening. A flexible grasping member is positioned within each of the pockets in the frame. A flexible grasping member is configured to engage a length of suture material to secure the length of suture material within the pocket. A flexible grasping member is a rubber insert. Slots are formed between the frame and sides of the rubber insert receipt of lengths of suture material. Each of the pockets includes a notch which is configured to receive a tooth of the rubber insert to secure the rubber insert within the pocket.

In one embodiment, the frame includes a concave portion surrounding the opening to guide surgical instruments towards the opening. The opening is defined by a rim of the frame and includes a plurality of cutouts for guiding lengths of suture material through the opening. The frame additionally includes a pair of wings to facilitate securing the frame to a surgical cannula.

There is further disclosed a suture management cap for use with a surgical cannula which generally includes an inner frame member defining a central opening and including a plurality of pockets and an outer frame member positionable over the inner frame member. Grasping members are positioned within the plurality of pockets and secured therein by the outer frame member. The grasping members are rubber inserts. The outer frame member includes a plurality of inwardly directed arms engageable with the grasping members to secure the grasping members within the pockets. Each of the rubber inserts includes a nose portion and each of the inwardly directed arms includes a pair of spaced apart teeth configured to surround the nose portion of the rubber insert. Slots are formed adjacent opposed sides of the inwardly directed arms and the rubber inserts include flexible blades configured to extend into the slots such that a length of suture material positioned within the slots is engaged by the flexible blades of the rubber inserts.

In one embodiment, the inner frame member includes a concave portion surrounding the opening. The opening is defined by a rim which includes a plurality of cutouts to maintain lengths of suture material and relationship. The outer frame member includes a pair of wings to facilitate securing the suture management cap to a surgical cannula.

In a specific embodiment, the inner frame member includes at least one vertically extending bar configured to engage the outer frame member to secure the inner frame member to the outer frame member.

There is also disclosed a suture management cap for use with a surgical cannula which generally includes a frame defining a longitudinal axis and a transverse axis. The frame has a central opening for passage of a surgical instrument and at least one grasping member formed on at least one edge thereof. The grasping members are configured and dimensioned to engage and secure a length of suture so that the suture can be selectively arranged in a predetermined configuration.

The grasping members are disposed on the distal edge of the frame along at least one of the longitudinal and transverse axes. Each grasping member includes a groove formed on the top surface of the suture management cap and at least one projection disposed adjacent the groove. The groove and projection are configured and dimensioned for orienting a length of suture relative to the edge of the frame. The distal ends of the groove and the projection are configured to facilitate vertical orientation of a length of suture.

The projection further includes at least one notch disposed on at least one edge thereof. Each notch is dimensioned to engage a portion of a length of suture.

In addition, the frame includes a concave portion surrounding the opening that is configured to guide surgical instruments towards the opening. The opening is defined by a rim disposed on the interior edge of the frame. The rim includes a plurality of cutouts dimensioned to guide lengths of suture towards at least one grasping member. The frame includes a pair of wings disposed at the distal ends thereof along the longitudinal axis. As such, the wings facilitate the securing of the frame to a surgical cannula.

In one embodiment of the suture management cap, a portion of at least one edge of the frame having the at least one grasping member formed thereon is configured and dimensioned to be selectively removable from the frame. In this embodiment, the selectively removable portion is made from a material softer than the frame material.

### DESCRIPTION OF THE DRAWINGS

Various embodiments of the presently disclosed suture management caps are disclosed herein with reference to the drawings, wherein:

FIG. 1 is a perspective view of a prior art cannula inserted in the body of a patient;

FIG. 2 is a side elevational view, partially shown in cross-section, of the prior art cannula of FIG. 1;

FIG. 3 is a perspective view of one embodiment of a suture management cap for use with a surgical cannula;

FIG. 4 is a top plan view of the suture management cap of FIG. 3;

FIG. 5 is a perspective view of a seal assembly for use with the disclosed suture management caps;

FIG. 6 is a top plan view of the seal assembly of FIG. 5;

FIG. 7 is a perspective view of a pair of seal members for use in the seal assembly of FIG. 5;

FIG. 8 is a perspective view of a guide member for use in a surgical cannula;

FIG. 9 is a top plan view of the guide member;

FIG. 10 is a perspective view of an alternative embodiment of a suture management cap;

FIG. 11 is a perspective view, with parts separated, of the suture management cap of FIG. 10;

FIG. 12 is a top plan view of the suture management cap of FIG. 10 with sutures in place;

FIG. 13 is an enlarged area of detail view of FIG. 12 with a suture in place;

FIG. 14 is a perspective view of a suture management cap with spring insert for use with a surgical cannula;

FIG. 15 is a perspective view, with parts separated, of the suture management cap of FIG. 14;

FIG. 16 is a top plan view of the suture management cap of FIG. 14 with sutures in place;

FIG. 17 is a perspective view of a three part suture management cap with rubber inserts for use with a surgical cannula;

FIG. 18 is a perspective view, with parts separated, of the suture management cap of FIG. 17;

FIG. 19 is a top plan view of the suture management cap of FIG. 17;

FIG. 20 is a perspective view of a two part suture management cap with rubber inserts for use with a surgical cannula;

FIG. 21 is a perspective view, with parts separated, of the suture management cap of FIG. 20;

FIG. 22 is a top view of the suture management cap of FIG. 20;

FIG. 23 is a perspective view of one embodiment of the suture management cap in accordance with the present disclosure;

FIG. 24 is an enlarged top plan view of the grasping members of the suture management cap of FIG. 23;

FIG. 25 is a perspective view of another embodiment of the suture management cap in accordance with the present disclosure; and

FIG. 26 is a perspective view, with parts separated, of yet another embodiment of the suture management cap in accordance with the present disclosure.

### DETAILED DESCRIPTION OF EMBODIMENTS

Embodiments of the presently disclosed suture management devices or caps will now be described in detail with reference to the drawings wherein like numerals designate identical or corresponding elements in each of the several views. As is common in the art, the term 'proximal" refers to that part or component closer to the user or operator, i.e. surgeon or physician, while the term "distal" refers to that part or component further away from the user.

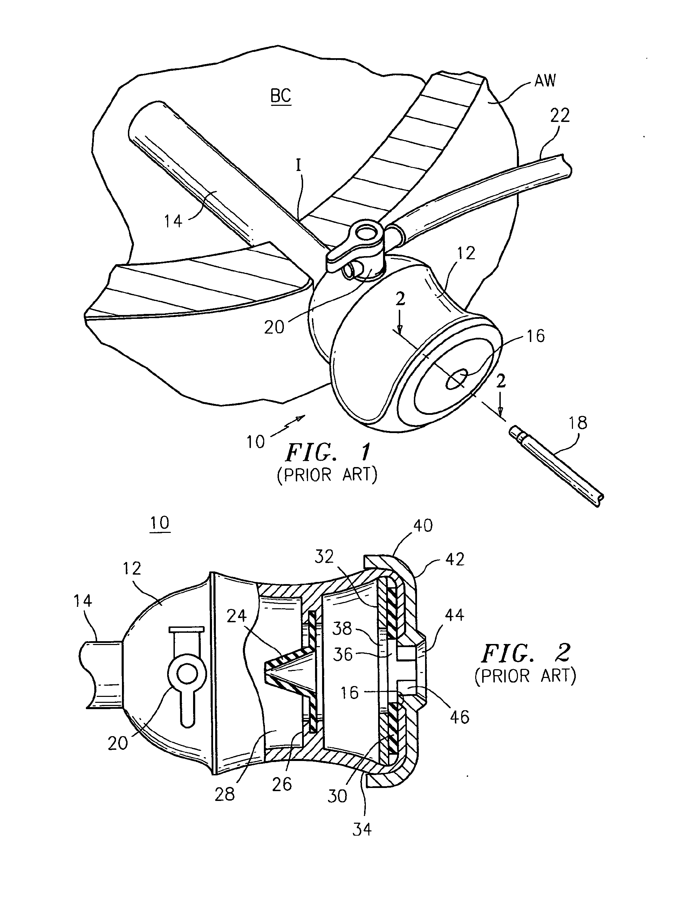

Referring initially to FIGS. 1 and 2, there is disclosed a prior art cannula port 10 for use during surgical procedures. Cannula port 10 generally includes a cannula housing 12 and an elongate tubular member 14 extending distally from housing 12. Elongate tubular member 14 is provided to extend through an incision I formed through body tissue, such as, for example, an abdominal wall AW and into a body cavity BC. Housing 12 includes a proximal opening 16 for receipt of a surgical instrument 18 therethrough. An insufflation valve 20 is provided on housing 12 and is connected to a source of insufflation fluid 24 for passage into body cavity BC to create a working space.

Referring specifically to FIG. 2, a duck bill valve 24 is mounted within a first flange 26 within housing 12 and serves to seal housing 12 against the escape of insufflation fluid in the absence of a surgical instrument. A proximal seal 30 is supported within a second flange 32 provided at a proximal end 34 of housing 12. Proximal seal 30 defines a seal opening 36 for receipt of, and sealing about, a surgical instrument inserted therethrough. It should be noted that, a flange opening 38 defined by second flange 32 is substantially larger in diameter than seal opening 36 formed through proximal seal 30.

In order to accommodate a variety of differing diameter surgical instruments, prior art cannula ports, such as prior art cannula port 10, may be provided with an auxiliary proximal cap 40. Proximal cap 40 generally includes a frame 42 configured to detachably engage proximal end 34 of housing 12. A tapered opening 44 is provided in frame 42 to guide surgical instruments towards an auxiliary seal 46 provided within a central opening 48 of frame 42. As noted here and above, auxiliary proximal cap 40 is provided to allow prior art cannula port 10 to accommodate differing diameter surgical instruments.

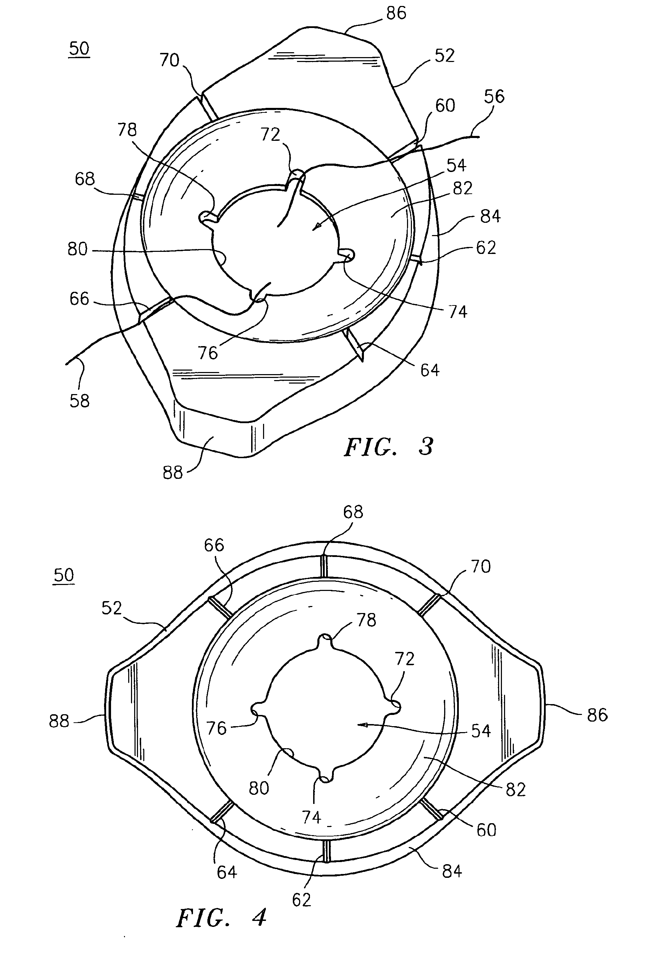

Referring now to FIGS. 3 and 4, there is disclosed a suture management cap 50 for use with a surgical cannula port, such as, for example cannula port 10 described herein above (FIG. 1). Suture management cap 50 is provided to control the spacing and tension on sutures, such as sutures 56 and 58, as they pass through suture management cap 50 and into cannula port 10. Suture management cap 50 generally includes a frame 52 defining a central opening 54. Central opening 54 overlies proximal opening 16 in housing 12 (FIG. 1) when suture management cap 50 is affixed to surgical cannula port 10. Suture management cap 50 may be releasably affixed to cannula port 10 by a variety of means, such as, for example, friction fit, snap fit, threading, etc.

In order to control the longitudinal movement of sutures through suture management cap 50, frame 52 is provided with a plurality of grasping members or v-shaped tapered grooves 60, 62, 64, 66, 68 and 70 provided on frame 52. Tapered grooves 60-70 are provided to receive sutures 56 and 58 such that sutures 56 and 58 may be wedged within grooves 60-70 in order to temporarily secure sutures 56 and 58 longitudinally with respect to frame 52.
In addition, a plurality of cutouts 72, 74, 76 and 78 are formed in rim 80 defining central opening 54. Cutouts 72-78 are provided to maintain sutures 56 and 58 in a spaced apart condition as they pass through central opening 54.

Frame 52 may be provided with a concave depression 82 extending between an outer edge 84 of frame 52 and central opening 54. Concave depression 82 facilitates guidance of surgical instruments towards central opening 54. Frame 52 may additionally be provided with a pair of wings 86 and 88 to facilitate manipulation of suture management cap 50 in order to secure suture management cap 50 with cannula port 10.

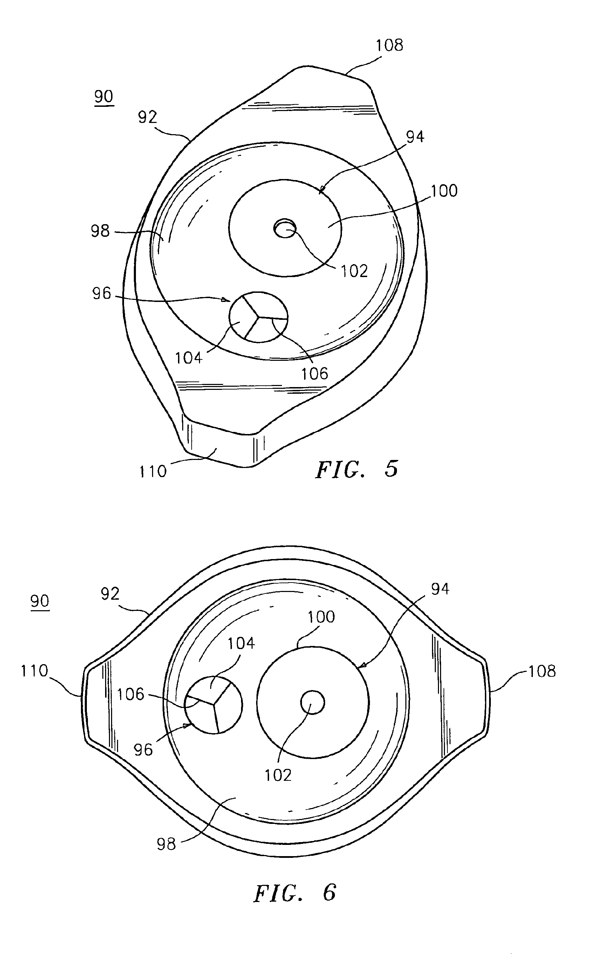

Referring for the moment to FIGS. 5 and 6, an auxiliary seal 90 is disclosed for use between the disclosed suture management caps and cannula port 10. Auxiliary seal 90 is provided to separate the passage of surgical instrument 18, described herein above, from sutures 56 and 58 as they pass into cannula port 10. Auxiliary seal 90 generally includes a seal body 92 defining a first port 94 and a second port 96. A concave depression 98 may be provided in seal body 92 to direct surgical instruments and sutures towards first and second ports 94 and 96, respectively. First port 94 is provided with a first seal 100 defining a first circular opening 102. First port 94 is provided to receive a surgical instrument through first circular opening 102 in first seal 100. Second port 96 includes a second seal 104 having a tri-part slit 106. Tri-part slit 106 is provided to receive and seal about surgical sutures passing therethrough. Seal body 92 may additionally be formed with wings 108 and 110 to conform to wings 86 and 88 formed on frame 52 of suture management cap 50.

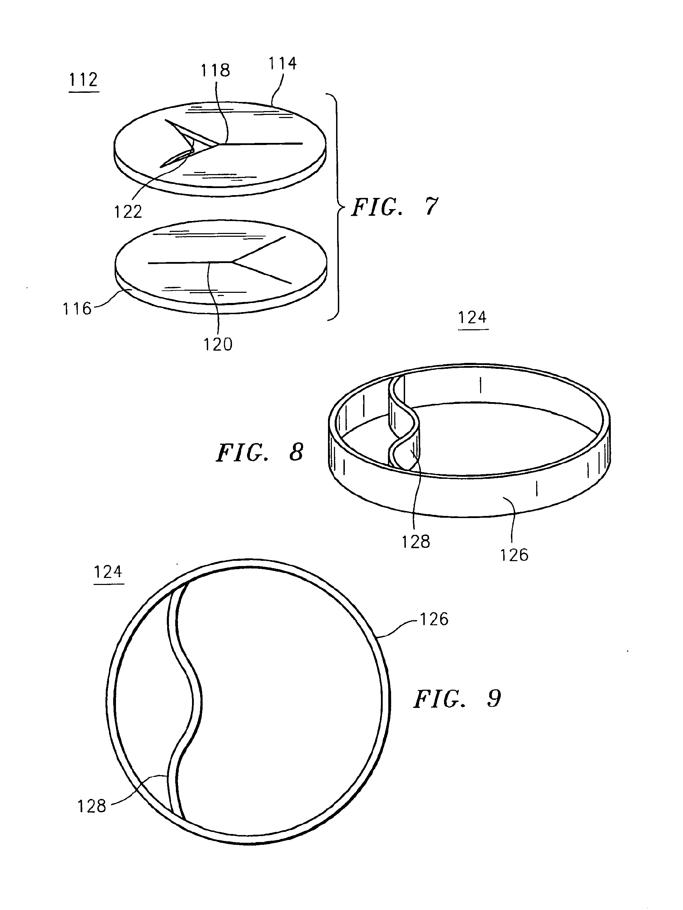

Referring to FIG. 7, there is disclosed an alternative embodiment of an auxiliary seal 112 for use in the disclosed suture management caps and cannula port 10. Auxiliary seal 112 includes a first disc 114 and a second disc 116. First and second discs 114 and 116 are formed of materials which will seal about instruments and sutures inserted therethrough. First disc 114 includes a first tri-part slit 118 and second disc 116 includes a second tri-part slit 120. First tri-part slit 118 includes a gap 122 for passage of surgical instruments therethrough. Second tri-part slit 120 is provided to receive and seal about surgical sutures inserted therethrough.

Referring now to FIGS. 8 and 9, in order to separately guide the passage of surgical instruments and sutures into housing 12 of cannula port 10, a guide 124 may be provided for positioning within either cannula port 10 or within the disclosed suture management caps. Guide 124 generally includes a circular frame 126 having an offset divider 128 formed therein. Surgical instruments and sutures pass around opposite sides of offset divider 128.

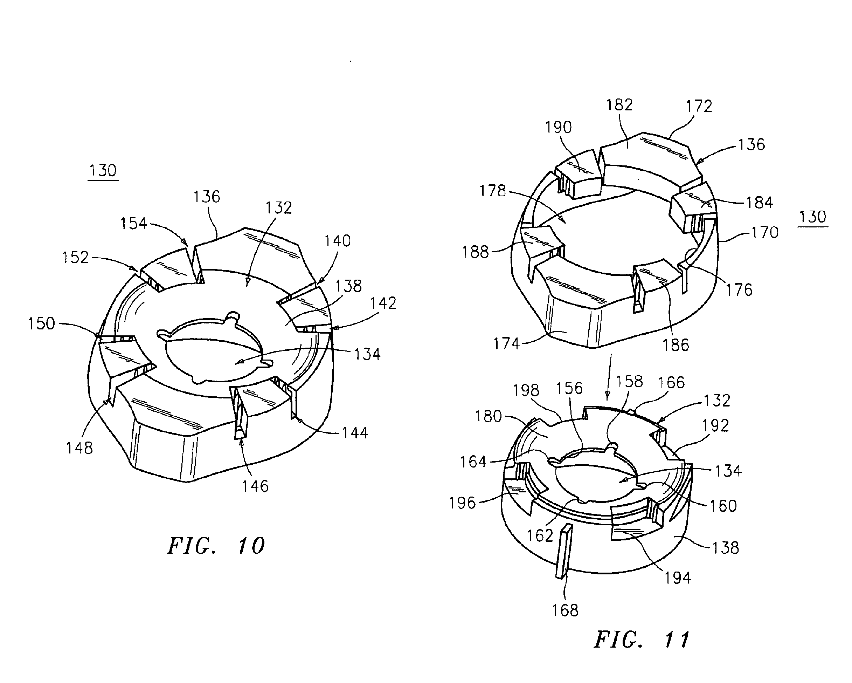

Referring to FIGS. 10-13, and initially with respect to FIGS. 10 and 11, there is disclosed an alternative embodiment of a suture management cap 130 for use with a surgical cannula. Suture management cap 130 generally includes an inner frame 132 defining an opening 134 for passage of surgical instruments and lengths of suture material and an outer frame 136 overlying inner frame 132. Inner frame 132 includes a circular inner body portion 138. A plurality of pairs of grasping members 140 and 142, 144 and 146, 148 and 150, and 152 and 154 are formed between inner frame 132 and outer frame 136. Grasping members 140-154 are provided to releasably secure a length of suture material temporarily positioned therein.

Referring to FIG. 11, inner body portion 138 includes a rim 156 defining opening 134. A plurality of cutouts 158, 160, 162 and 160 are provided along rim 156 to guide lengths of suture material into a surgical cannula used with suture management cap 130. A pair of bars 166 and 168 are provided on inner body portion 138 to engage corresponding components in the outer frame 136 (not shown) in order to secure inner frame 132 within outer frame 136. Similar to suture management cap 50 described herein above, outer frame 136 has an outer body portion 170 which includes a pair of wings 172 and 174 to facilitate attaching suture management cap 130 to a surgical cannula.

Outer body portion 170 includes a discontinuous rim 176 which defines an outer frame opening 178 through which a portion of inner body portion 138 projects such that an upper surface 180 of inner body portion 138 is flush with an upper surface 182 of outer body portion 170. As shown, upper surface 180 of inner body portion 138 is concave so as to direct surgical instruments towards opening 134.

Outer body portion 170 further includes a plurality of inwardly projecting arms 184, 186, 188 and 190 which are configured to be received in respective recesses or pockets 192, 194, 196 and 198 formed in inner body portion 138. Grasping members 140-154 are formed between side surfaces of the inwardly projecting arms and the respective pockets in a matter described in more detail hereinbelow.

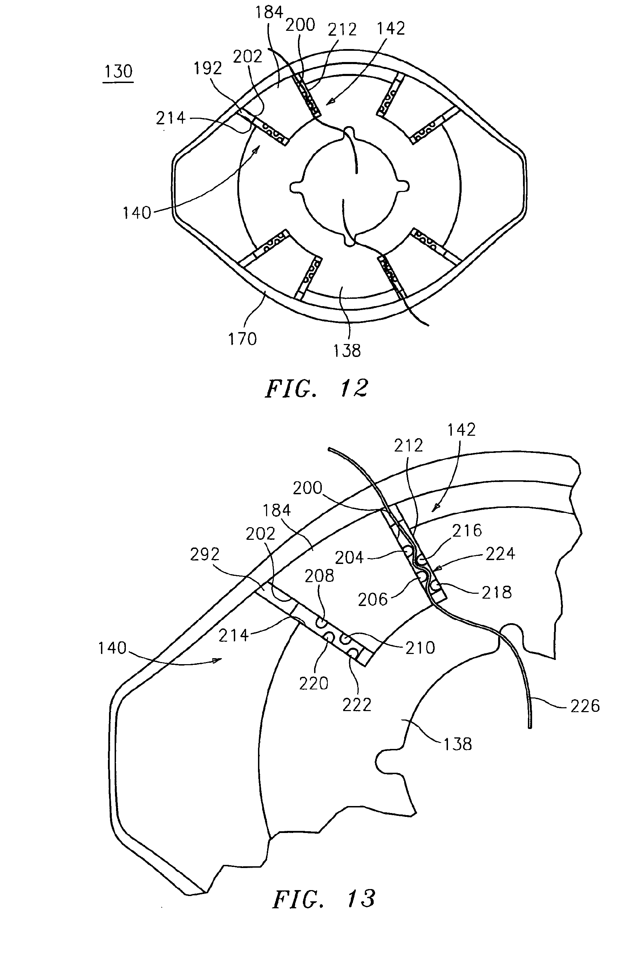

Referring now to FIGS. 12-13, it should be noted that, while the following description is given with regard to grasping members 140 and 142, the structure and operation of the remaining grasping members 144-154 are identical to that described with respect to grasping members 140 and 142. Inwardly projecting arm 184 includes a pair of side surfaces 200 and 202. With reference to FIG. 13, side surface 200 includes a pair of vertically extending ribs 204 and 206. Similarly, side surface 202 includes a pair of vertically extending ribs 208 and 210. Likewise, pocket 192 is defined by a pair of side surfaces 212 and 214. Side surface 212 includes a pair of vertically extending ribs 216 and 218 and side surface 214 includes a pair of vertically extending ribs 220 and 222.

As shown, ribs 204 and 206 projecting from side surface 200 of inwardly projecting arm 184 are horizontally offset from, and interleaved with, ribs 216 and 218 projecting from side surface 212 of inner body portion 138. Likewise, ribs 208 and 210 projecting from side surface 202 of inwardly projecting arm 184 are also horizontally offset from, and interleaved with, ribs 220 and 222 projecting from side surface 214 of inner body portion 138. Thus, grasping member 140 is formed from the interleaved positioning of ribs 208, 210 with ribs 220 and 222. Likewise, grasping member 142 is formed from the interleaved positioning of ribs 216, 218 with ribs 204 and 206.

As used herein, the term "interleaved" refers to the projection of ribs on one side of a gap, for example, gap 224 defined between opposed side surface 202 of inwardly projecting arm 184 and side surface 214 of inner body member 138, into the spaces defined between ribs 208, 210, 220 and 222 positioned on opposite sides of gap 224.

Thus, the provision of opposing pairs of vertically extending ribs formed in opposed side surfaces of inner body member 138 and a respective opposed side surfaces of an inwardly projecting arm function to form grasping members 140-146. As specifically shown in FIG. 13, when a length of suture material 226 is passed through grasping member 142, length of suture material 226 is temporarily secured therein by the interleaved relationship of ribs 216, 218 with ribs 204 and 206.

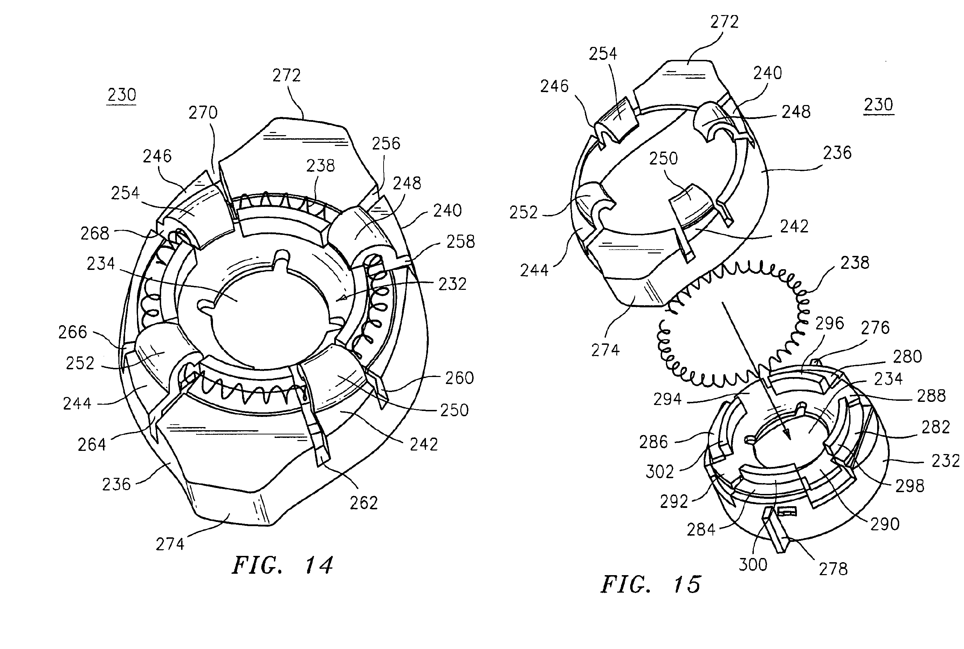

Referring to FIGS. 14-16, and initially with regard to FIG. 14, there is disclosed a further alternative embodiment of a suture management cap 234 use with a surgical cannula. Suture management cap 230 generally includes an inner frame member 232 defining an opening 234 for passage of surgical instruments and lengths of suture material therethrough. Suture management cap 230 additionally includes an outer frame member 236 and a grasping member or length of coil spring 238 positioned between inner frame member 232 and outer frame member 236. In order to secure coil spring 238 between inner frame member 232 and outer frame member 236, outer frame member 236 includes a plurality of inwardly projecting arms 240, 242, 244 and 246.

Inwardly projecting arms 240, 242, 244 and 246 terminate in respective hooks 248, 250, 252 and 254 which are configured to overlie coil spring 238 and hold coil spring 238 on inner frame member 232 as described hereinbelow. Slots are provided about the periphery of outer frame member 236 to guide lengths of suture material through outer frame member 236 and towards coil spring 238. Specifically, slots 256 and 258 are formed on opposed sides of inwardly projecting arm 240. Similarly, respective pairs of slots 260 and 262, 264 and 266, and 268 and 270 are formed on opposed sides of inwardly projecting arms 242, 244 and 246. Similar to those suture management caps described herein above, outer frame member 236 includes a pair of opposed wings 272 and 274 to facilitate securing suture management cap 230 to a surgical cannula.

Referring now to FIG. 15, in order to secure inner frame member 232 to outer frame member 236, inner frame member 232 is provided with a pair of vertically extending bars 276 and 278 which are configured to engage corresponding structure on outer frame member 236 (not shown) in friction fit fashion. As noted herein above, coil spring 238 rests on inner frame member 232 and is held in place by outer frame member 236. Inner frame member 232 includes a plurality of circumferential shelf portions 230, 282, 284 and 286 which are provided to support coil spring 238. Respective pockets 288, 290, 292 and 294 are formed between shelf portions 280-286 to receive, and provide clearance for, inwardly projecting arms 240-246 of outer frame member 236. In order to further support coil spring 238 between outer frame member 236 and inner frame member 232, shelf portions 280, 282, 284 and 286 are provided with respective wall portions 296, 298, 300 and 302.

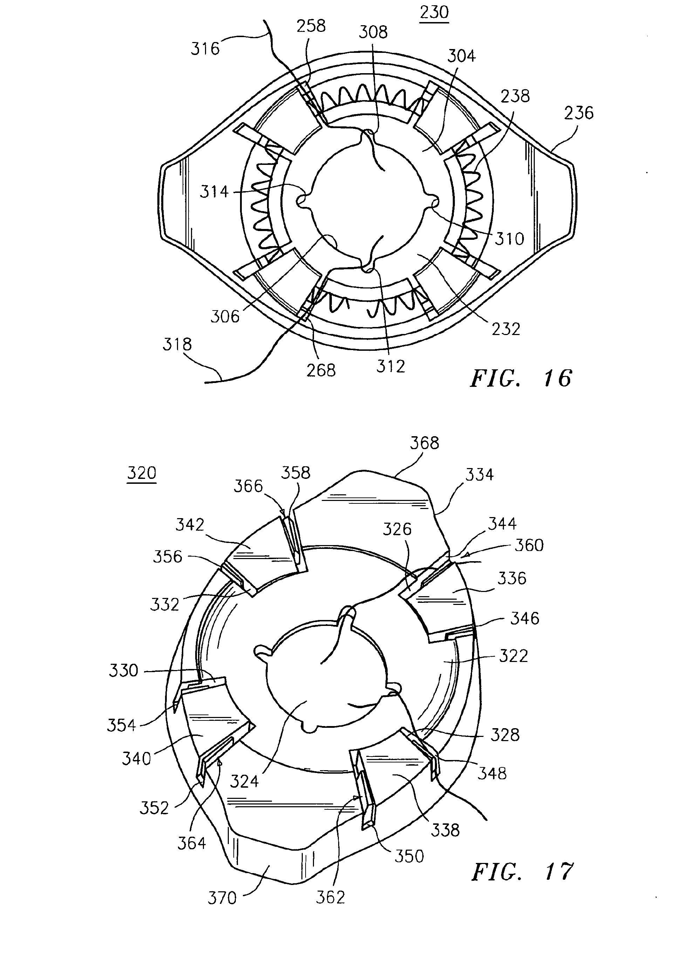

As best shown in FIG. 16, inner frame member 236 includes a concave inner surface 304 surrounding opening 234. Specifically, a rim 306 formed in inner surface 304 defines opening 234. In order to separate and guide lengths of suture material through opening 234, a plurality of cutouts 308, 310, 312 and 314 are formed within rim 306.

In use, lengths of suture material 316 and 318 are passed over coil spring 238 specifically, for example, length of suture material 316 extends through slot 258 and is retained therein by the bias of coil spring 238. Length of suture material 316 then passes over concave inner surface 304 of the inner member 232 and extends through cut out 308 which guides length of suture material 316 towards a surgical cannula. Similarly, length of suture material 318 extends through slot 268 and across coil spring 238 and through cut out 312. Thus, coil spring 238, in conjunction with slots 256 and 268, forms a grasping member for temporarily securing lengths of suture material 316 and 318. Additionally, cutouts 308 and 312 function as guide members to guide lengths of suture material 316 and 318 through opening 234 and towards a surgical cannula.

It should be noted that, as discussed herein above, the remaining disclosed slots, in conjunction with coil spring 238, function identically to that described herein above and serve to form grasping members for temporarily securing lengths of suture material to suture management cap 230.

Referring now to FIGS. 17-19, and initially with regard to FIG. 17, there is disclosed a three-part suture management cap 320 for use with a surgical cannula. Suture management cap 320 generally includes an inner frame member 322 defining a first opening 324 for receipt of surgical instruments therethrough. A plurality of circumferentially spaced pockets 326, 328, 330, and 332 are formed in inner frame member 322. An outer frame member 334 is provided to surround inner frame member 322 and includes a plurality of inwardly directed arms 336, 338, 340 and 342. Pairs of slots 344 and 346, 348 and 350, 352 and 354 and 356 and 358 are respectively provided on opposite sides of inwardly directed arms 336, 338, 340 and 342. In order to secure lengths of suture material within the slots, suture management 230 is further provided with grasping members or rubber inserts 360, 362, 364 and 366 which are positioned within pockets 326, 328, 330 and 332 in inner frame member 322 respectively. It should be noted that, while the disclosed inserts are described as being formed of rubber, other materials such as, for example, plastics, ceramics, etc. may be provided to extend into a respective slots as described hereinbelow in order to temporarily secure a length of suture within the respective slot.

Similar to the suture management caps described herein above, suture management cap 230 includes a pair of wings 368 and 370 extending from outer frame member 334 to facilitate securing suture management cap 230 to a surgical cannula.

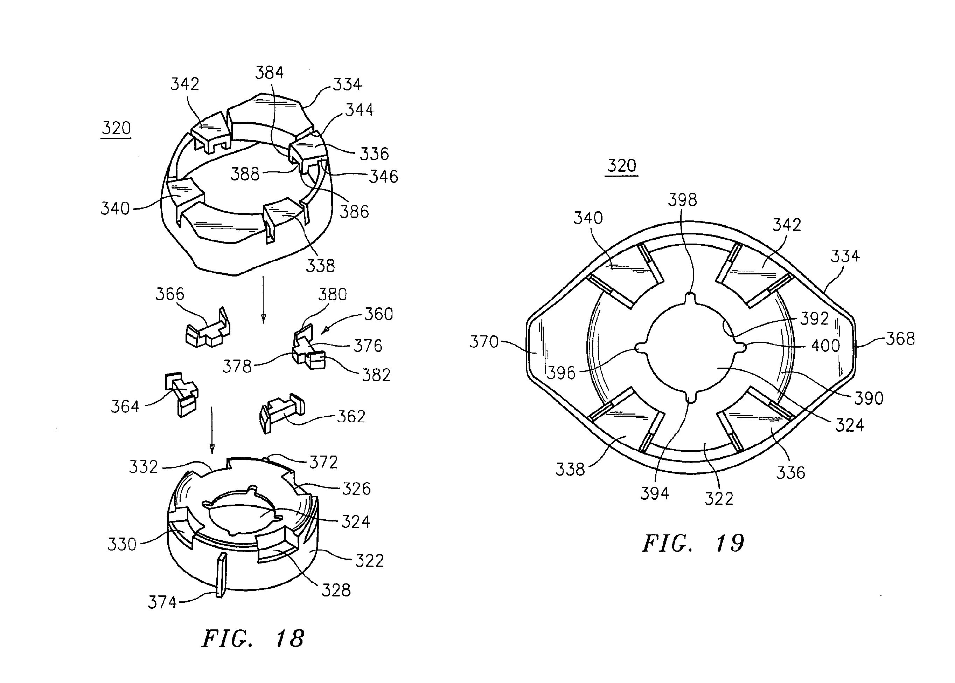

Referring now to FIG. 18, inner frame member 322 is provided with a pair of vertically extending bars 372 and 374 to facilitate securing inner frame member 322 to outer frame member 334. The structure of one of the rubber inserts, for example, rubber insert 360, will now be described along with inwardly projecting arm 336. It should be noted that, however, the remaining inwardly projecting arms 338, 340 and 342 along with the remaining rubber inserts 362, 364 and 366 are structurally and functionally identical to rubber inserts 360 and inwardly projecting arm 336 discussed hereinbelow.

As noted herein above, rubber insert 360, positioned within pocket 326 of inner frame member 322, along with slots 344 and 346 formed in outer frame member 334 forms a pair of grasping members to temporarily secure a length of suture material. Rubber insert 360 includes a T-shaped base 376 having an inwardly directed nose 378. A pair of upright blades 380 and 382 extends upwardly from base 376. Upright blades 380 and 382 are configured to project into slots 344 and 346 in outer frame member 334. Thus, a length of suture material placed within one of slots 344 and 346 is secured therein by engagement with one of upright blades 380 and 382.

In order to retain rubber insert 360 within slot 326 inwardly projecting arm 336 of outer frame member 334 includes a pair of downwardly projecting teeth 384 and 386 which define a gap 388 therebetween and are configured to straddle or surround nose 378 of rubber insert 360.

Referring to FIG. 19, inner frame member 322 includes a concave portion 390 surrounding opening 324. A rim 392 is formed within concave portion 390 and defines opening 324. In order to maintain several lengths of suture material in spaced apart relation as they extend through opening 324, rim 392 is provided with a plurality of cutouts 394, 396, 398 and 400.

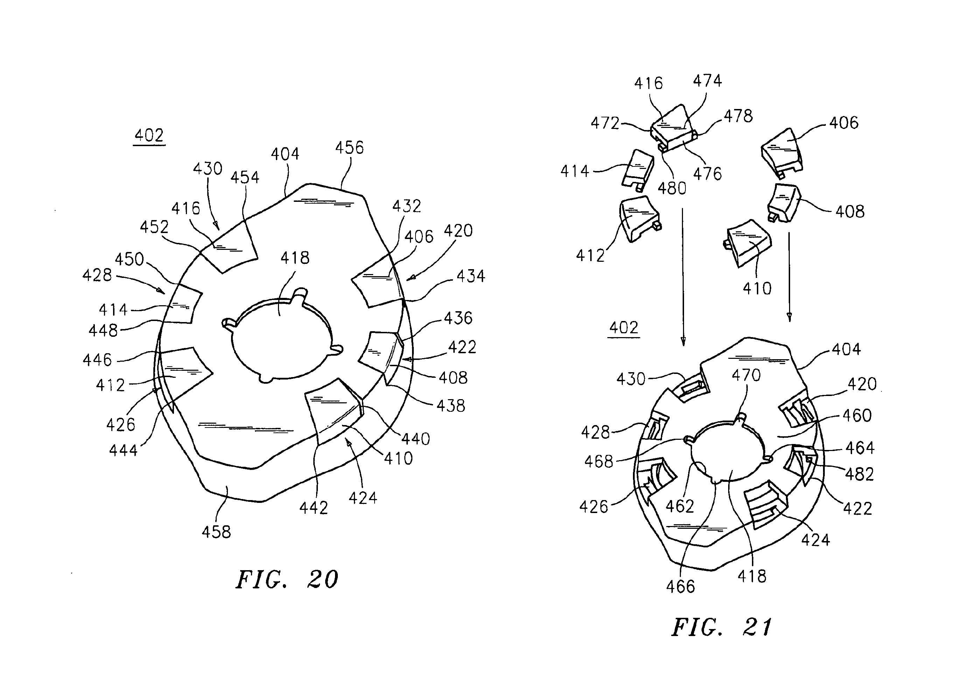

Referring now to FIGS. 20-22, and initially with regard to FIG. 20, there is disclosed a further alternative embodiment of a two-part suture management cap 402 for use with a surgical cannula. Suture management cap 402 generally includes a frame member 404 having a plurality of flexible, rubber inserts 406, 408, 410, 412, 414 and 416 positioned therein. Frame member 404 includes a central opening 418 to receive surgical instruments therethrough. Frame member 404 includes a plurality of circumferentially spaced pockets 420, 422, 424, 426, 428 and 430 for respective receipt of rubber inserts 406, 408, 410, 412, 414 and 416. Pairs of slots 432 and 434, 436 and 438, 440 and 442, 444 and 446, 448 and 450 and 452 and 454 are thus formed on opposite sides of respective inserts 406, 408, 410, 412, 414 and 416 for receipt and securement of lengths of suture materials inserted therethrough. Similar to those suture management caps described herein above, frame member 404 includes a pair of wings 456 and 458 to facilitate securing suture management cap 402 to a surgical cannula.

Referring now to FIG. 21, frame member 404 includes a concave portion 460 having a circular rim 462 defining opening 418. A plurality of cutouts 464, 466, and 468 and 470 are provided in circular rim 462 in order to guide lengths of suture material into opening 418 and maintain the lengths of suture material in spaced apart relation.

The structure of one of the rubber inserts such as, for example, rubber insert 416 will now be described. It should be noted that, however, the structure and function of the remaining rubber inserts 406, 408, 410, 412 and 414 are identical to that described with respect to rubber insert 416. As noted herein above, rubber insert 416 is constrained within pocket 430 on frame member 404. Rubber insert 416 generally includes a proximal base 472 having an inward arm 474 extending therefrom. A downward leg 476 extends from inward arm 474 and terminates in a pair of laterally extending side teeth 478 and 480. Side teeth 478 and 482 are configured to engage respective notches (for example notch 482 as best shown in pocket 422) to retain rubber insert 416 within pocket 430.

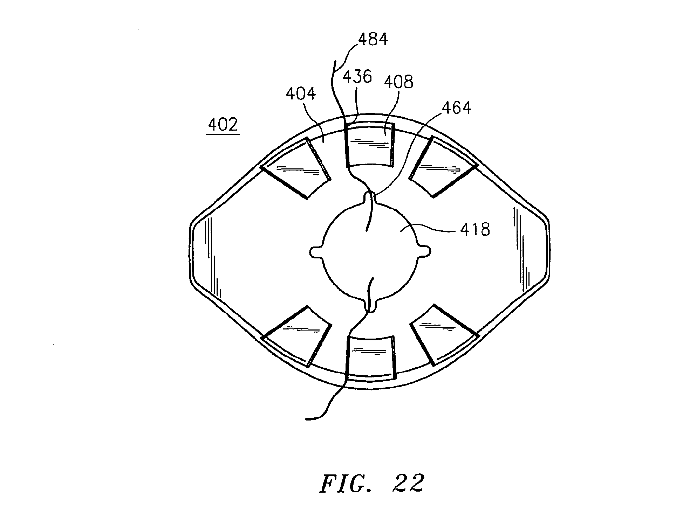

Referring out of FIG. 22, in use, a length of suture materials such as, for example, suture material 484 is passed through slot 436 and secured therein by engagement between rubber insert 408 and frame member 404. Suture material 484 is guided through cutout 464 into central opening 418 for passage down into a surgical cannula.

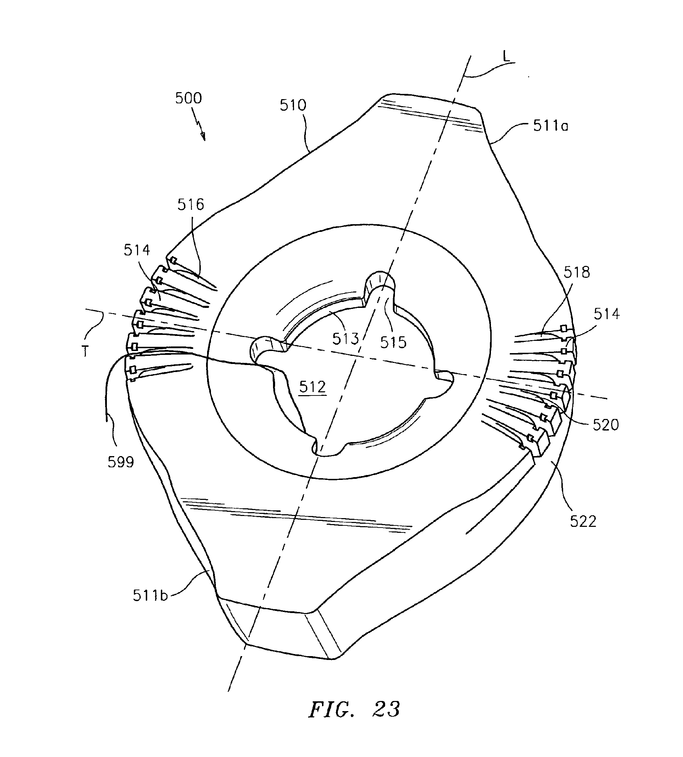

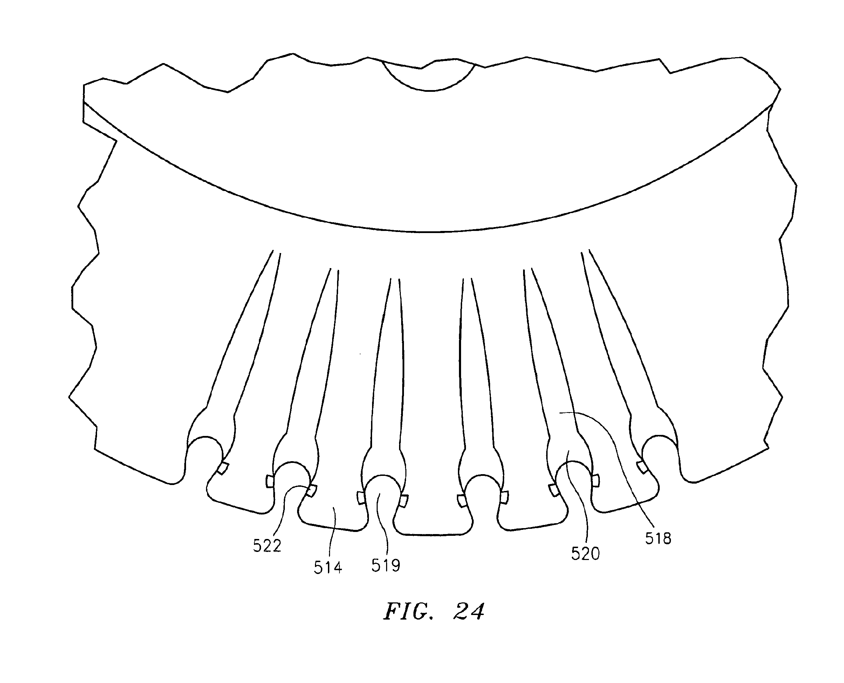

Referring now to FIGS. 23 and 24, there is disclosed another embodiment of a suture management cap 500 for use with a surgical cannula. The suture management cap 500 generally includes a frame 510 defining a longitudinal axis L and a transverse axis T. The frame 510 has a central opening 512 for passage of a surgical instrument. In addition, the frame 510 includes at least one grasping member 514 formed on at least one edge thereof. The frame 510 further includes a concave portion 516 surrounding the opening 512. The concave portion 516 is configured and dimensioned to guide surgical instruments towards the opening 512. The frame 510 further includes a pair of wings 511a, 511b disposed at the distal ends of the frame 510 along the longitudinal axis. The wings 511a, 511b facilitate the securing of the frame 510 to a surgical cannula.

Each grasping member 514 is configured and dimensioned to engage and secure at least one length of suture 599, whereby each grasping member 514 enables the at least one length of suture 599 to be selectively arranged in a predetermined configuration. In this embodiment, each grasping member 514 is disposed on a distal edge of the frame 510 along the transverse axis T. As shown in FIG. 23, at least one grasping member 514 is distally disposed substantially along each of the opposing radial edges and substantially disposed about the transverse axis T.

Each grasping member 514 includes a groove 518 formed on the top surface of the suture management cap 500. The grasping member 514 further includes at least one projection 520 disposed adjacent the at least one groove 518. Each groove 518 and each projection 520 are configured and dimensioned for orienting at least one length of suture 599 relative to at least one edge of the frame 510. The distal end of each groove 518 and each projection 520 defining a space 519 therebetween. The space 519 is configured and dimensioned to facilitate the vertical orientation of the length of suture 599. Each projection 520 further includes at least one notch 522 disposed on the distal edge thereof. Each notch 522 is configured and dimensioned to engage a portion of a length of suture 599 for tying a portion of each length of suture 599 to the distal end of each projection 520.

In this embodiment, the opening 512 is defined by a rim 513 disposed on the interior edge of the frame 510. The rim 513 includes a plurality of cutouts 515, each of which is configured and dimensioned to guide lengths of suture 599 towards the at least one grasping member 514. The cutouts 515 also allow instruments to be inserted or removed without engaging or potentially damaging sutures 599 passing through opening 512.

In use, lengths of suture material 599 are passed over the concave portion 516 of the frame 510. The lengths of suture material 599 extend through the opening 512 and into the grasping members 514. The grasping members 514 guide the lengths of suture material 599 so that the lengths of suture material 599 can be arranged in a predetermined configuration.

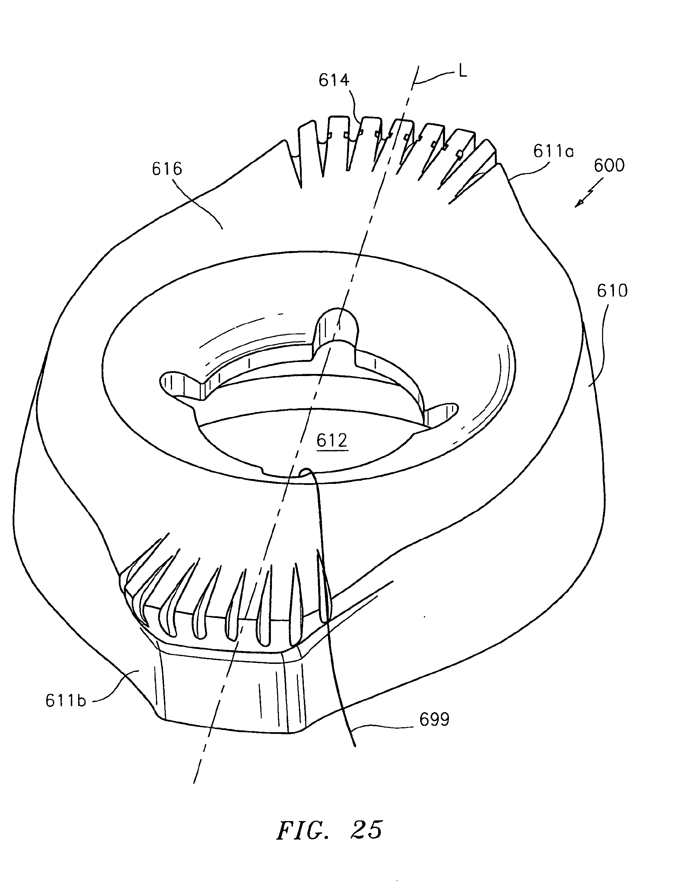

Referring now to FIG. 25, there is disclosed yet another embodiment of a suture management cap 600 for use with a surgical cannula. The suture management cap 600 is substantially similar to the suture management cap of 500. However, the suture management cap 600 includes at least one grasping member 614 disposed on at least one edge along the longitudinal axis L. As illustrated in FIG. 25, a plurality of grasping members 614 are disposed on each of the distal most edges along the longitudinal axis L. In particular, the grasping members 614 are shown disposed on each of the wings 611a 611b.

In use, lengths of suture material 699 are passed over the concave portion 616 of the frame 610. The lengths of suture material 699 extend through the opening 612 and into the grasping members 614. The grasping members 614 guide the lengths of suture material 699 so that the lengths of suture material 699 can be arranged in a predetermined configuration.

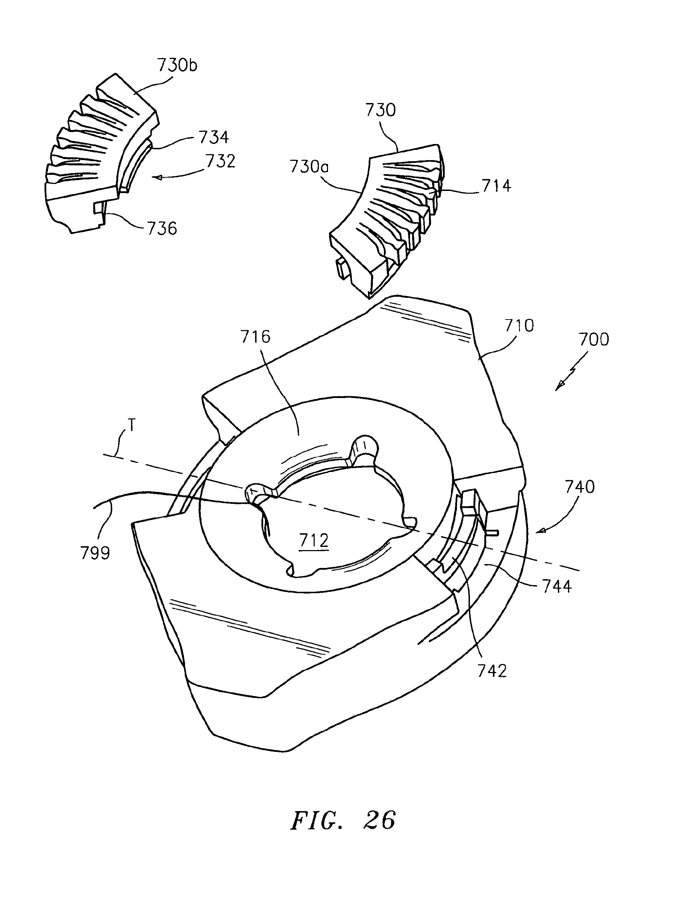

Referring now to FIG. 26, there is disclosed a still further embodiment of a suture management cap 700 for use with a surgical cannula. The suture management cap 700 is substantially similar to the suture management cap of 500. However, the suture management cap 700 includes a portion of at least one of the edges that is configured and dimensioned to be selectively removable from the frame 710. In this embodiment, at least one grasping member 714 is formed on the selectively removable portion 730.

As shown in FIG. 26, a pair of selectively removable portions 730a, 730b is disposed on the distal edges along transverse axis T. It is envisioned that the selectively removable portion 730 is made from a material softer than the frame 710 material. Each selectively removable portion 730 includes an attaching feature 732 having a first and second connecting member 734, 736. In addition, the frame 710 further includes an attaching seat 740 formed on at least one of the distal edges thereof. The attaching seat 740 has an upper and lower ledge 742, 744. The first connecting member 734 is configured and dimensioned to removably attach to the upper ledge 742. The second connecting member 736 is configured and dimensioned to removably attach to the lower ledge 744.

In use, lengths of suture material 799 are passed over the concave portion 716 of the frame 710. The lengths of suture material 799 extend through the opening 712 and into the grasping members 714. The grasping members 714 guide lengths of suture material 799 so that the lengths of suture material 799 can be arranged in a predetermined configuration. In this embodiment, the selectively removable portion 730 can be interchanged with a plurality of other selectively removable portions 730. For example, one selectively removable portion having two grasping members 714 can be interchanged with another selectively removable portion having ten grasping members 714, depending on the particular need or procedure.

It will be understood that various modifications may be made to the embodiments disclosed herein. For example each embodiment of the disclosed suture management caps may include more or less than the disclosed number of grasping members. Further, as noted above, various known methods may be employed to temporarily secure the disclosed suture management caps to a surgical cannula. Therefore, the above description should not be construed as limiting, but merely as exemplifications of particular embodiments. Those skilled in the art will envision other modifications within the scope and spirit of the claims appended hereto.

## Claims

1. A suture management cap for use with a surgical cannula comprising:
a frame defining a longitudinal axis and a transverse axis, the frame having a central opening for passage of a surgical instrument, the frame including at least one grasping member formed on at least one edge thereof, each grasping member is configured and dimensioned to engage and secure at least one length of suture, whereby each grasping member enables the at least one length of suture to be selectively arranged in a predetermined configuration.

2. A suture management cap according to claim 1 wherein the at least one grasping member is disposed substantially along the at least one edge, the at least one edge substantially disposed about at least one of the longitudinal and transverse axes.

3. A suture management cap according to claim 1 or claim 2 wherein the at least one grasping member further includes at least one groove formed on the top surface of the suture management cap and at least one projection disposed adjacent the at least one groove, the at least one groove and the at least one projection configured and dimensioned for orienting the at least one length of suture relative to the at least one edge of the frame.

4. A suture management cap according to claim 3 further comprising at least one space defined between the at least one projection and the at least one groove, wherein the space is configured and dimensioned to facilitate vertical orientation of the at least one length of suture.

5. A suture management cap according to claim 3 or claim 4 wherein the at least one projection further includes at least one notch disposed on at least one edge thereof, the at least one notch configured and dimensioned to engage a portion of the at least one length of suture.

6. A suture management cap according to any preceding claim, wherein the frame includes a concave portion surrounding the opening, the concave portion configured and dimensioned to guide surgical instruments towards the opening.

7. A suture management cap according to any preceding claim, wherein the opening is defined by a rim disposed on the interior edge of the frame, wherein the rim includes a plurality of cutouts configured and dimensioned to guide lengths of suture towards the at least one grasping member.

8. A suture management cap according to any preceding claim, wherein the frame includes a pair of wings disposed at the distal ends of the frame along the longitudinal axis to facilitate the securing of the frame to a surgical cannula.

9. A suture management cap according to any preceding claim, wherein a portion of the at least one edge having the at least one grasping member formed thereon is configured and dimensioned to be selectively removable from the frame.

10. A suture management cap according to claim 9, wherein the portion of the at least one edge having the grasping member formed thereon is made from a material softer than the frame material.
